# EUROPEAN PATENT SPECIFICATION

(11) **EP 3 265 778 B1**
(45) Date of publication and mention of the grant of the patent: **01.12.2021**
(21) Application number: 16711060.0
(22) Date of filing: 26.02.2016
(51) Int. Cl.: G01N 17/00

(54) **METHOD FOR MONITORING MICROBIOLOGICALLY INDUCED CORROSION ON METAL SURFACES**
VERFAHREN ZUR ÜBERWACHUNG VON MIKROBIOLOGISCH INDUZIERTER KORROSION AUF METALLOBERFLÄCHEN
PROCÉDÉ DE SURVEILLANCE DE LA CORROSION INDUITE MICROBIOLOGIQUEMENT SUR DES SURFACES MÉTALLIQUES

(30) Priority: 06.03.2015 US 201562129071 P
(43) Date of publication of application: 10.01.2018
(73) Proprietor: Rohm and Haas Company, Philadelphia, PA 19106 (US); Nutrition & Biosciences USA 1, LLC, Rochester, NY 14623 (US)
(72) Inventor: ARUMUGAM, Selvanathan, Collegeville, PA 19426 (US); ASHMORE, John W., Lansdale, PA 19446 (US); GHOSH, Tirthankar, Collegeville, PA 19426 (US); MCGINLEY, Heather R., Buffalo Grove, IL 60089 (US)
(74) Representative: Houghton, Mark Phillip
(86) International application number: PCT/US2016/019671
(87) International publication number: WO 2016/144565

(56) References cited:
- WO-A1-2012/149487
- US-A1- 2005 151 971
- M. FISCHER ET AL: "Fluorescence-Based Quasicontinuous and In Situ Monitoring of Biofilm Formation Dynamics in Natural Marine Environments", APPLIED AND ENVIRONMENTAL MICROBIOLOGY, vol. 80, no. 12, 11 April 2014 (2014-04-11), pages 3721-3728, XP055270861, US ISSN: 0099-2240, DOI: 10.1128/AEM.00298-14

## Description

### Background

This invention relates generally to a method for monitoring microbiologically induced corrosion on metal surfaces.

The use of silicates to inhibit corrosion of carbon steel surfaces is known. For example, US3580934 describes this use of silicate salts. However, the prior art does not disclose a method for monitoring microbiologically induced corrosion (MIC) on metal surfaces.

US 2005/0151971 discloses a real-time biofilm monitoring system. Fischer et al. disclose "Fluorescence-Based Quasicontinuous and In Situ Monitoring of Biofilm Formation Dynamics in Natural Marine Environments" in Applied and Environmental Microbiology, June 2014, Volume 80, Number 12, pages 3721-3728.

### Statement of Invention

The present invention is directed to a method for monitoring microbiologically induced corrosion on metal surfaces in contact with a fluid; said method comprising adding to the fluid a silanol compound or silanol compound precursor having a group detectible by ultraviolet spectrophotometry or fluorescence spectroscopy, or which is isotopically enriched, and monitoring the level of free silanol in the fluid released from the metal surface as a biofilm forms by detecting the uv-active or fluorescent group, or isotopically enriched atom, by known spectroscopic techniques.

The present invention is further directed to a method as hereinbefore defined, said method comprising adding to the fluid a silicate compound which is isotopically enriched.

### Detailed Description

All percentages are weight percentages (wt%), and all temperatures are in °C, unless otherwise indicated. All experimental work was performed at room temperature ("RT"), i.e., in the range from 20-25°C, unless specified otherwise. Concentrations in parts-per-million ("ppm") are on a weight/weight basis.

As used herein, the term "metal surface" refers to the surface of a metal (preferably iron or steel), although this surface may have a natural coating of metal oxide.

As used herein, the term "silanol" refers to a compound having a hydroxyl group attached directly to a silicon atom. Preferably, the silanol has from one to three hydroxyl groups. Preferably, the group detectible by ultraviolet spectrophotometry or fluorescence spectroscopy is an organic group, i.e., one having carbon and hydrogen atoms and having no transition metal atoms, preferably one having only carbon, hydrogen, oxygen, sulfur and nitrogen atoms. In one preferred embodiment of the invention, the silanol is generated in situ in the fluid from a silanol compound precursor by adding a compound which hydrolyzes to a silanol (e.g., a trialkoxysilane or trichlorosilane). Preferably, the silanol compound precursor has formula XSi(G)₃ wherein G represents C₁-C₈ alkyl, C₁-C₈ alkoxy, chloro or a combination thereof provided that there is at least one alkoxy or chloro present; preferably X'CH₂Si(G)₃ preferably X'CH₂Si(OR)₃; wherein X or X' represents a uv-active or fluorescent group and R is C₁-C₈ alkyl. Preferably, R is C₁-C₄ alkyl, preferably methyl. In one preferred embodiment, the silanol compound has the formula X'CH₂Si(R)₂OH, wherein R is C₁-C₈ alkyl; the silanol compound precursor for this compound has a chloro or alkoxy group in place of the hydroxyl group.

The silanol or silanol compound precursor is added to the fluid and the level of free silanol compound in the fluid is monitored. Preferably, the level of silanol is monitored by detecting the uv-active or fluorescent group by known spectroscopic techniques.

Preferably, silanol or silanol compound precursor is added to the fluid to produce an initial silanol concentration of at least 10 ppm, preferably at least 30 ppm, preferably at least 50 ppm, preferably at least 70 ppm, preferably at least 90 ppm; preferably no more than 2 wt%, preferably no more than 1 wt%, preferably no more than 0.5 wt%, preferably no more than 0.1 wt%, preferably no more than 300 ppm, preferably no more than 250 ppm, preferably no more than 200 ppm. When the fluid is non-aqueous, e.g., oil or natural gas, preferably the initial silanol concentration is no more than 300 ppm, preferably no more than 250 ppm, preferably no more than 200 ppm.

Preferably, a uv-active group has a uv λₘₐₓ of at least 220 nm, preferably at least 230 nm, preferably at least 240 nm, preferably at least 250 nm; preferably no higher than 400 nm, preferably no higher than 370 nm, preferably no higher than 350 nm. Preferably, a fluorescent group has an excitation maximum from 220 to 650 nm and an emission maximum from 250 to 800 nm. A silanol or silicate which is isotopically enriched has at least one atom which is enriched in one isotope of that atom beyond the natural abundance level. The atom may be silicon, oxygen, hydrogen or another atom present in an organic substituent group of a silanol. An isotopically enriched organic substituent group preferably has the formula X'CH₂ wherein X' is a group having from one to eighteen carbon atoms as well as hydrogen atoms, and which may also have oxygen, nitrogen and/or sulfur atoms. Preferably, X' has no metal atoms. Preferably, X' contains no atoms other than carbon, hydrogen, oxygen, nitrogen and sulfur. Silanols or silicates which are isotopically enriched may be detected by mass spectrometry.

Preferably, the concentration of silanol or silicate in the fluid at various times is determined by the appropriate method, depending on the nature of the group attached to the silanol or the isotopic enrichment of the silicate.

Silanols having uv-active groups include, e.g., bis(4-triethoxysilylpropoxy-3-methoxyphenyl)-1,6-heptadiene-3,5-dione; 3-(2,4-dinitrophenylamino)propyltriethoxysilane; 2-hydroxy-4-(3-methyldiethoxysilylpropoxy)diphenyl ketone; 2-hydroxy-4-(3-triethoxysilylpropoxy)diphenyl ketone; 7-triethoxysilylpropoxy-5-hydroxyflavone; 2-(2-triethoxysilylpropoxy-5-methylphenyl)benzotriazole; 3-(triethoxysilylpropyl)-p-nitrobenzamide and (R)-N-triethoxysilylpropyl-O-quinine-urethane.

Silanols having fluorescent groups include, e.g., O-4-methylcoumarin-N-[3-(triethoxysilyl)propyl]carbamate and N-(triethoxysilylpropyl)dansylamide Preferably, uv-active or fluorescent groups are attached to the silanol silicon atom via a carbon chain, preferably -(CH₂)ₙ-, where n is from one to six, preferably from one to four.

The silanol is released as biofilm forms on the metal surface. The concentration of silanol in the fluid correlates with growth of biofilm and the rate of increase of the concentration correlates with the rate of growth. Growth of corrosive biofilm is known to correlate with the MIC process.

Preferably, the fluid has a water content of at least 0.01 wt%, preferably at least 0.1 wt%, preferably at least 0.5 wt%, preferably at least 1 wt%. Preferably, the fluid is crude oil, natural gas, water or an aqueous solution having at least 75% water (preferably at least 90%)

### Examples

Typical MIC Detection Procedure: Sterile anaerobic test vials were prepared. Serum bottles (10 mL bottle) were filled with culture media containing synthetic sea salts (35 g/L), glucose (1 g/L), sodium lactate (0.5 g/L), yeast extract (0.1 g/L), and sodium acetate (0.05 g/L). Peg-shaped carbon steel coupons were added to each one of the bottle. Serum bottles were sealed and autoclaved. For series 3 experiments, silica-treated coupons were used. Next, serum bottles were inoculated with SRB and APB at 1:100 dilutions from saturated cultures. For Series 1 experiments, soluble silicates (130-175 ppm) were introduced at this point. All samples were incubated at RT. Aliquots from the samples were drawn at different time points before complete formation of biofilm, centrifuged and prepared it for ICP analysis. The samples were left at RT to allow biofilm formation. When there was black color or precipitate in the coupon-containing inoculated samples, which is indicative of biofilm formation, more aliquots were drawn at different time points, centrifuged and prepared it for ICP analysis. The carbon steel surface was analyzed by XPS to determine the surface bound silica. For Series 2 experiments, soluble silicates (130-175 ppm) were introduced after the biofilm formation. The silica concentration in the supernatant is correlated the rate of biofilm action on metal bound silica. The amount of silica in supernatant after 24 hrs of introduction indicates the amount of silica that is immobilized on the coupon surface. The immobilized silica concentration is indicator of metal surface area available, in other words the extent of biofilm at the point of introduction.

Silica treatment of carbon steel coupons: A standard solution of soluble silicates of concentration 130-175 ppm was prepared. A freshly polished carbon steel coupon was immersed in the silicate solution and incubated at RT for 24 h. The metal coupon was removed from the silicate solution and washed with 5mL DI water thrice and the washings were combined with the incubate solution. The combined incubate solution was analyzed by ICP to determine the silica left over in the solution. From the silica remaining in the supernatant, the silica adsorbed to the surface was estimated.

**Testing the influence other chemical and physical triggers on the silica release in MIC samples:** The influence of infinite dilution, acid pH, basic pH, corrosion inhibitor such as alkybenzyl ammonium chloride, corroding chemicals such as hydrogen sulfide were monitored over time by following the procedure described below. Carbon steel coupons pretreated with silica and the physically adsorbed silica was washed with DI water. The silica treated coupons were then placed in vial containing (1) DI water, (2) Instant Ocean (culture Media) (3) 0.1M HCl (pH 1), (4) 1mM NaOH (pH 11), (5) corrosion inhibitor - alkybenzyl ammonium chloride (100 ppm in DI water), (6) hydrogen sulfide (150 µM) and (7) sterile water containing SRB bacteria. The supernatant and carbon steel coupon were respectively analyzed by ICP and XPS to determine released silica and the silica remained bound to coupon.

**Table 1. Silica release profile Series 1 - Silica introduced along with SRB to untreated coupon**

| Sample | Incubation Time (days) | Concentration of Silica released (ppm) |
|---|---|---|
| 1 | 1 | 2.99 |
| 2 | 4 | 12.84 |
| 3 | 7 | 42.66 |
| 4 | 21 | 94.23 |
| 5 | 28 | 118.1 |

**Table 2. Silica release profile Series 2 - Silica introduced after biofilm formation**

| Sample | Incubation Time (days) | Concentration of Silica released (ppm) |
|---|---|---|
| 1 | 7 | 52.71 |
| 2 | 8 | 69.42 |
| 3 | 10 | 83.78 |
| 4 | 21 | 107.2 |
| 5 | 28 | 126.5 |

**Table 3. Silica release profile Series 3- Coupons were pretreated with silica 24h prior to SRB addition**

| Sample | Incubation Time (days) | Concentration of Silica released (ppm) |
|---|---|---|
| 1 | 1 | 5 |
| 2 | 3 | 7.27 |
| 3 | 7 | 31.28 |
| 4 | 14 | 38.13 |
| 5 | 21 | 66.76 |
| 6 | 28 | 72.16 |

**Table 4. Comparison of Silica release profile in presence of various chemical and biological triggers**

| Sample | Trigger | Concentration of Silica released after 3 weeks incubation (%) |
|---|---|---|
| 1 | Dilution (DI water) | 3.7 |
| 2 | Instant Ocean (culture media) | 5.6 |
| 3 | Acid (0.1M HCl) | 4.9 |
| 4 | Base (0.001M NaOH) | 6.1 |
| 5 | Alkybenzyl ammonium chloride (100 ppm) | 5.3 |
| 6 | Hydrogen Sulfide (150 µM) | 7.1 |
| 7 | Sterile water containing SRM (biofilm) | 89.6 |

**Table 5. Comparison of Silica release profile in presence of various chemical and biological triggers**

| Sample | Trigger | Δ Silica Concentration on carbon steel coupon (%) |
|---|---|---|
| 1 | Dilution (DI water) | 3 |
| 2 | Instant Ocean (culture media) | 0.0 |
| 3 | Acid (0.1M HCl) | 0.0 |
| 4 | Base (0.001M NaOH) | 11.2 |
| 5 | Alkybenzyl ammonium chloride (100 ppm) | 0.0 |
| 6 | Hydrogen Sulfide (150 µM) | 2.9 |
| 7 | Sterile water containing SRM (biofilm) | 100.0 |

The results demonstrate that the ferrosilicate/silica layer is stable at infinite dilution, acidic pH and basic pH. Exposure to chemicals such alkylbenzylammonium salts and hydrogen sulfide does not trigger the release of silicates from the carbon steel surface. However once biofilm starts growing on the surface, the chemically tethered silica is released back into the aqueous stream. The concentration of soluble silicates present in the aqueous stream serves as an indicator of available metal surface area and the rate at which silicates are released upon the action of corrosive biofilms serve as an indicator for occurrence of the MIC process at the surface.

## Claims

1. A method for monitoring microbiologically induced corrosion on metal surfaces in contact with a fluid; said method comprising adding to the fluid a silanol compound or silanol compound precursor having a group detectible by ultraviolet spectrophotometry or fluorescence spectroscopy, or which is isotopically enriched, and monitoring the level of free silanol in the fluid released from the metal surface as a biofilm forms by detecting the uv-active or fluorescent group, or isotopically enriched atom, by known spectroscopic techniques.

2. The method of claim 1 in which said silanol or silanol compound precursor is added to the fluid to produce an initial silanol concentration from 10 ppm to 2 wt%.

3. The method of claim 2 in which the silanol compound precursor has formula XSi(G)₃, wherein G represents C₁-C₈ alkyl, C₁-C₈ alkoxy, chloro or a combination thereof provided that there is at least one alkoxy or chloro present; and wherein X represents a uv-active or fluorescent group.

4. The method of claim 3 in which a silanol compound precursor is added to the fluid.

5. The method of claim 4 in which the fluid has a water content from 0.01 to 100 wt%.

6. The method of claim 5 in which the silanol compound precursor has a group detectible by ultraviolet spectrophotometry or fluorescence spectroscopy

7. The method of claim 6 in which the fluid is crude oil having at least 0.05 wt% water.

8. The method of claim 7 in which the fluid is natural gas having at least 0.05 wt% water.

9. The method of claim 1, said method comprising adding to the fluid a silicate compound which is isotopically enriched.

10. The method of claim 9 further comprising monitoring a level of isotopically enriched silicate compound in the fluid.

## Patentansprüche

1. Verfahren zur Überwachung von mikrobiologisch induzierter Korrosion an Metalloberflächen in Kontakt mit einem Fluid; wobei das Verfahren Hinzufügen einer Silanolverbindung oder eines Silanolverbindungsvorläufers zu dem Fluid umfasst, welche(r) eine mittels Ultraviolett-Spektrofotometrie oder Fluoreszenz-Spektroskopie detektierbare Gruppe aufweist, oder welche istotopisch angereichert ist, und Überwachen des Spiegels an freiem Silanol in dem aus der Metalloberfläche freigesetzten Fluid, während sich ein Biofilm bildet, durch Detektieren der UV-aktiven oder fluoreszierenden Gruppe, oder des isotopisch angereicherten Atoms, durch bekannte spektroskopische Techniken.

2. Verfahren nach Anspruch 1, wobei das Silanol oder der Silanolverbindungsvorläufer zu dem Fluid hinzugefügt wird, um eine anfängliche Silanolkonzentration von 10 ppm bis 2 Gewichtsprozent zu erzeugen.

3. Verfahren nach Anspruch 2, wobei der Silanolverbindungsvorläufer die Formel XSi(G)₃ aufweist, wobei G C₁-C₈ Alkyl, C₁-C₈ Alkoxy, Chlor oder eine Kombination hieraus darstellt, vorausgesetzt, dass mindestens ein Alkoxy oder ein Chlor vorhanden ist; und wobei X eine UV-aktive oder fluoreszierende Gruppe darstellt.

4. Verfahren nach Anspruch 3, wobei ein Silanolverbindungsvorläufer zu dem Fluid hinzugefügt wird.

5. Verfahren nach Anspruch 4, wobei das Fluid einen Wassergehalt von 0,01 bis 100 Gewichtsprozent aufweist.

6. Verfahren nach Anspruch 5, wobei der Silanolverbindungsvorläufer eine mittels Ultraviolett-Spektrofotometrie oder Fluoreszenz-Spektroskopie detektierbare Gruppe aufweist.

7. Verfahren nach Anspruch 6, wobei das Fluid Rohöl ist, welches mindestens 0,05 Gewichtsprozent Wasser aufweist.

8. Verfahren nach Anspruch 7, wobei das Fluid Erdgas ist, welches mindestens 0,05 Gewichtsprozent Wasser aufweist.

9. Verfahren nach Anspruch 1, wobei das Verfahren Hinzufügen einer isotopisch angereicherten Silicatverbindung zu dem Fluid umfasst.

10. Verfahren nach Anspruch 9, ferner umfassend Überwachen eines Spiegels einer isotoptisch angereicherten Silicatverbindung in dem Fluid.

## Revendications

1. Procédé pour surveiller la corrosion induite microbiologiquement sur des surfaces métalliques en contact avec un fluide ; ledit procédé comprenant l'ajout au fluide d'un composé de silanol ou d'un précurseur de composé de silanol qui comporte un groupe qui peut être détecté par spectrophotométrie par ultraviolets ou par spectroscopie par fluorescence, ou qui est enrichi isotopiquement, et la surveillance du niveau de silanol libre dans le fluide qui est libéré depuis la surface métallique lorsqu'un biofilm est formé en détectant le groupe réagissant aux UV ou fluorescent, ou l'atome enrichi isotopiquement, au moyen de techniques spectroscopiques connues.

2. Procédé selon la revendication 1, dans lequel ledit silanol ou ledit précurseur de composé de silanol est ajouté au fluide pour produire une concentration initiale en silanol de 10 ppm à 2 % en poids.

3. Procédé selon la revendication 2, dans lequel le précurseur de composé de silanol présente la formule XSi(G)₃, dans laquelle G représente C₁-C₈ alkyle, C₁-C₈ alcoxy, chlore ou une combinaison de ceux-ci à condition qu'il y ait au moins un alcoxy ou un chlore présent ; et dans lequel X représente un groupe réagissant aux UV ou fluorescent.

4. Procédé selon la revendication 3, dans lequel un précurseur de composé de silanol est ajouté au fluide.

5. Procédé selon la revendication 4, dans lequel le fluide présente une teneur en eau de 0,01 à 100 % en poids.

6. Procédé selon la revendication 5, dans lequel le précurseur de composé de silanol comporte un groupe qui peut être détecté par spectrophotométrie par ultraviolets ou par spectroscopie par fluorescence.

7. Procédé selon la revendication 6, dans lequel le fluide est du pétrole brut qui comporte au moins 0,05 % en poids d'eau.

8. Procédé selon la revendication 7, dans lequel le fluide est du gaz naturel qui comporte au moins 0,05 % en poids d'eau.

9. Procédé selon la revendication 1, ledit procédé comprenant l'ajout au fluide d'un composé de silicate qui est enrichi isotopiquement.

10. Procédé selon la revendication 9, comprenant en outre la surveillance d'un niveau d'un composé de silicate enrichi isotopiquement dans le fluide.
